## Europäisches Patentamt

## European Patent Office

## Office européen des brevets

(19)

(11) Publication number: **0 136 829**
A2

(12)

# EUROPEAN PATENT APPLICATION

(21) Application number: **84305996.5**

(22) Date of filing: **31.08.84**

(51) Int. Cl.⁴: **C 12 N 15/00**
C 12 P 21/02, C 12 N 1/20
//(C12N1/20, C12R1:19)

(30) Priority: **02.09.83 JP 162337/83**

(43) Date of publication of application:
**10.04.85 Bulletin 85/15**

(84) Designated Contracting States:
**AT BE CH DE FR GB IT LI LU NL SE**

(71) Applicant: **SUNTORY KABUSHIKI KAISHA, also known as SUNTORY LTD.**
**1-40, Dojimahama 2-chome**
**Kita-ku Osaka530(JP)**

(72) Inventor: **Nomura, Midori**
**20-31, Habikino 4-chome**
**Habikino Osaka 583(JP)**

(72) Inventor: **Ohsuye, Kazuhiro**
**18-7, Inaba-cho**
**Ibaraki Osaka 567(JP)**

(72) Inventor: **Tanaka, Shoji**
**8-46, Minamikaneden-cho 1-chome**
**Suita Osaka 564(JP)**

(74) Representative: **Raynor, John et al,**
**W.H.Beck, Greener & Co 7 Stone Buildings Lincoln's Inn**
**London WC2A 3SZ(GB)**

(54) Improved expression vector and use thereof.

(57) A novel *Escherichia coli* plasmid is described. The plasmid has, between the Shine-Dalgarno sequence and the translation start codon, a base sequence different from the corresponding, originally occurring region in the β-galactosidase operon and contains, upstream therefrom, the region of a hybrid promoter between the *Escherichia coli trp* promoter and the *lac* promoter and, at an entirely independent site, a *lac* promoter repressor gene. Employing an *Escherichia coli* transformant carrying the plasmid, a useful polypeptide such as h-IFN-γ can be produced in high yield.

EP 0 136 829 A2

## Improved expression vector and use thereof

This invention relates to Escherichia coli hybrid plasmid vectors improved so as to enable high efficiency production of useful polypeptides and to the use of said vectors.

Heretofore, a number of useful polypeptides have been produced in microbial or higher animal or plant cells as hosts with the aid of the so-called recombinant DNA technology (hereinafter, rDNA technology), and general techniques therefore can be said to have already been established. However, there still remains a room for improvement with respect to hosts and vector systems, among others, for enabling efficient and economical production in actual commercialization or application.

A variety of factors can be taken into consideration in relation to efficient production of useful polypeptides in relation to efficient production of useful polypeptides using the rDNA technology. As for expression vectors to be carried by Escherichia coli, for instance, such factors may include the kind of promoter involved in the promotion of transcription of the exogeneous gene desired to be expressed, the

base sequence of the so-called Shine-Dalgarno sequence (hereinafter, SD sequence) involved in complementary binding of the 3' terminal sequence of 16S ribosome RNA to mRNA and the distance between said sequence and the translation start codon (ATG), and the copy number of plasmid vector. However, for certain polypeptides desired to be produced in high efficiency, there are no established rules known yet between these factors and the productivity of said peptides. For instance, a certain expression vector capable of causing high expression of a certain exogeneous gene cannot always bring about high expression of another exogeneous gene. In cultivating transformants, an expression vector capable of inducing efficient introduction and expression of an exogeneous gene, if available, will also be important in actual high production of a useful polypeptide. For example, an expression vector with which transformant cell growth and plasmid DNA replication proceed normally without expression of an exogeneous gene inserted in said vector under certain cultivation conditions but the transcription of said exogeneous gene is rapidly accelerated under different cultivation conditions will be important, because the formation of a polypeptide, which is often toxic to the host (transformant), as the product of an exogeneous gene at the initial stage of cell growth may possibly inhibit the growth of the host microorganism and as a result cause decrease in productivity.

From the above viewpoint, various modifications of expression vectors have so far been attempted. For instance, there are known a hybrid promoter between the *Escherichia* *coli* tryptophan (trp) promoter and the lactose UV5 promoter (tac promoter; cf. H. A. de Boer et al., Proc. Natl. Acad. Sci. USA, 50, 21, 1983 and Japanese Patent Application Kokai Tokkyo Koho No. 194790/1982), the so-called runaway plasmid the copy number of which rapidly increases at elevated temperatures (cf. B. E. Uhlin et al., Gene, 6, 91, 1979 and Jpanese national publication of patent application under PCT No. 50057/1980), and a plasmid modified in the SD sequence or in the distance between the SD sequence and the translation startpoint to thereby increase the translation efficiency (Japanese Patent Application Kokai Tokkyo Koho No. 50893/1980, Japanese Patent Application No. 132524/1983).

The present inventors paid their attention to the above points and succeeded to modify in various ways the vector pGIF54 for exogeneous gene expression that had already been constructed (essentially the same plasmid as pGIF4 disclosed in Japanese Patent Application No. 86180/1982; an *Escherichia* *coli* strain transformed with said plasmid has been named SBMG105 and deposited with the Fermentation Research Institute, Agency of Industrial Science and Technology, Ministry of International Trade and Industry, Japan, under the deposit numbers FERM P-6522 and FERM BP-282) in an attempt to construct Escherichia coli plasmid vectors capable of realizing high-yield and high-efficiency production of useful polypeptides.

The details are described in the following.

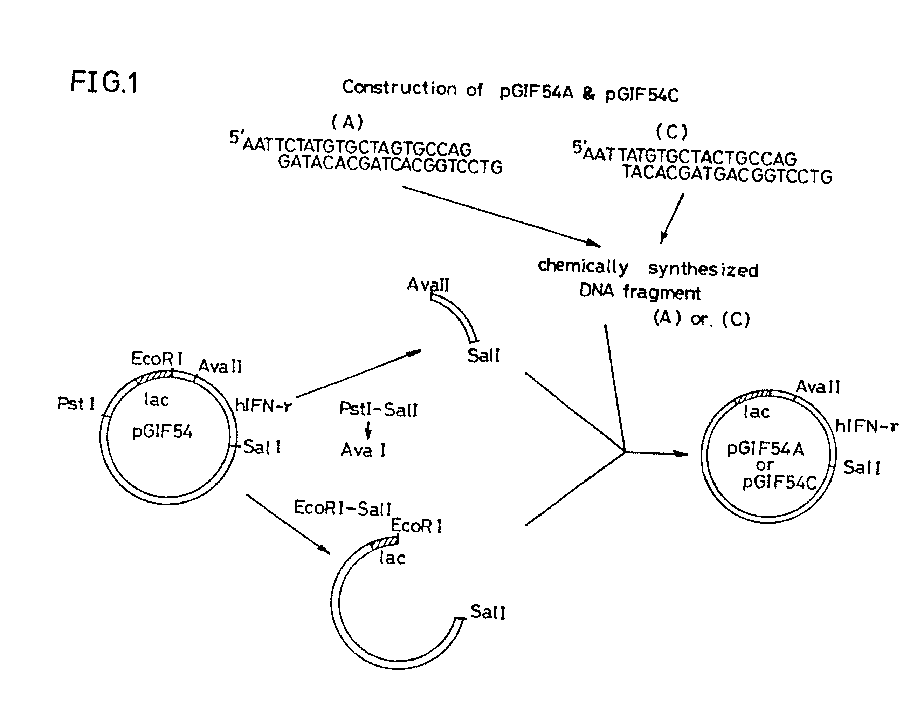

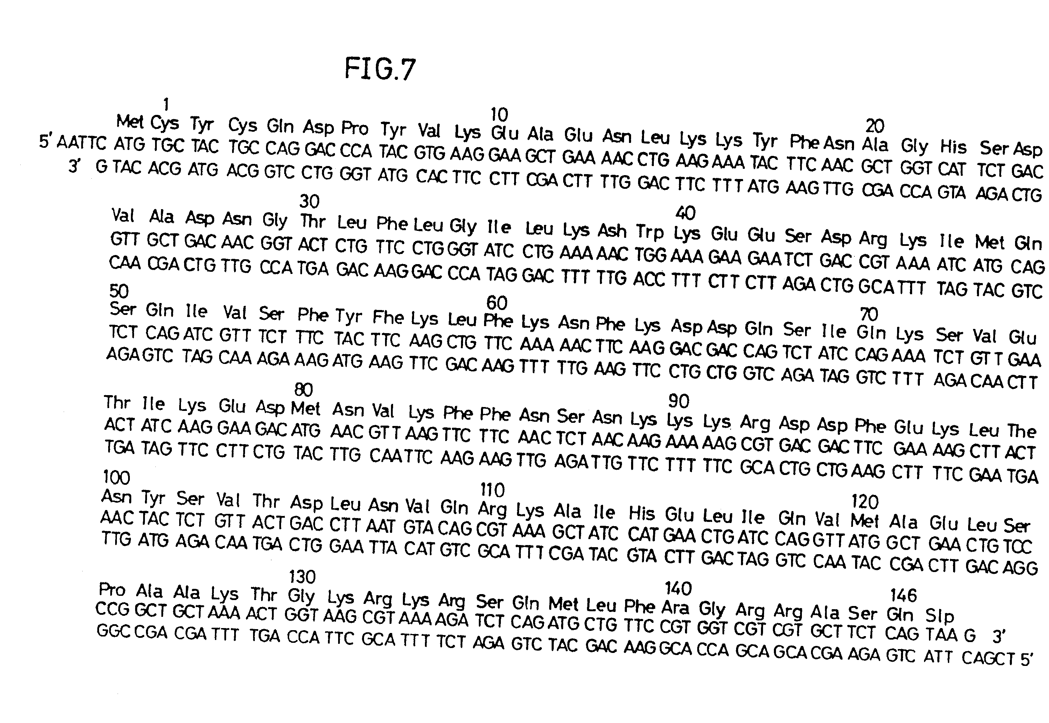

As already mentioned, the SD sequence or the distance between the SD sequence and the translation start codon (ATG) has an influence on the efficiency of heterologous polypeptides production. Therefore, the present inventors first attempted to modify the distance between the SD sequence and translation start codon ATG residing on the plasmid pGIF54. The plasmid pGIF54 is a human gamma-interferon (hereinafter, hIFN-γ) expression vector containing a DNA fragment which comprises a gene (cf. Fig. 7) chemically synthesized and coding for a polypeptide having the amino acid sequence of hIFN-γ as connected downstream from the β-galactosidase gene-derived lac UV5 promoter (cf., as to said plasmid, Japanese Patent Application No. 86180/1982). In said plasmid, the distance between the SD sequence and the translation start codon (ATG) for hIFN-γ is longer by 3 base pairs as compared with the original lac promotor and β-galactosidase case. In view of the secondary structure of mRNA, the present inventors expected that modification of the above- mentioned distance might lead to increase in the efficiency of translation of the hIFN-γ polypeptide. Therefore, they constructed a plasmid, pGIF54A, with said distance increased by one base and a plasmid, pGIF54C, with said distance reduced by one base and studied the production of hIFN-γ in Escherichia coli strains transformed with the respective plasmids. The modification of the distance between the SD sequence and ATG was accomplished by replacing the EcoRI-AvaII fragment of pGIF54 with either of two chemically synthesized DNA fragments (A) and (C) differing in length, as illustrated in Fig. 1. The 5' end of the upper strand of each of the chemically synthesized double-stranded DNA fragments consists of AATT and constitutes an EcoRI cohesive end. However, since the base subsequent to AATT is A in the DNA fragment (C), the plasmid pGIF54C with said fragment inserted therein no more has the EcoRI site. (AATTC would constitute an EcoRI recognition site.)

In Escherichia coli strains transformed with these plasmids, the hIFN-γ activity was determined and found to be about 3 times and about 8 times with pGIF54A and pGIF54C, respectively, as compared with the case of pGIF54. It was thus found that the modification of the distance between the SD sequence and ATG greatly influences the productivity of the heterologous peptide (hIFN-γ).

For further increase in productivity, the present inventors then modified the plasmid pGIF54C by modification of the promoter (introduction of the $\underline{tac}$ promoter), introduction of the $\underline{lac}$ promoter repressor gene $i^q$ (cf. The Operon, pp. 31-88, editors: J. H. Miller and W. S. Reznikoff, Cold Spring Harbor Laboratory, 1978) and introduction of the runaway plasmid (B. E. Uhlin et al., Gene, $\underline{6}$, 91, 1979) DNA.

The $\underline{tac}$ promoter is a hybrid promoter having the -35 region for the $\underline{Escherichia\ coli\ trp}$ promoter and the Pribnow box and the subsequent region of the $\underline{Escherichia\ coli\ lac}$ promoter (cf. H. A. de Boer et al., Proc. Natl. Acad. Sci. USA, $\underline{80}$, 21, 1983). Characteristic features of this hybrid promoter are that it has very strong promoter activity and that the expression of structural genes which are downstream therefrom is controlled by the $\underline{lac}$ promoter and repressor. The latter means that the addition of an inducer such as IPTG (isopropyl-β-D-thiogalactoside) can activate this promoter. Whereas the construction of the $\underline{tac}$ promoter has been described in the reference cited above, the present inventors independently constructed the $\underline{tac}$ promoter and further constructed a plasmid, ptacGIF54C, which is capable of high production of useful polypeptides under the control of said promoter.

Since it does not contain the $\underline{lac}$ promoter repressor gene $\underline{laci}^q$, the plasmid ptacGIF54C can cause constitutional expression of an exogenerous gene (the hIFN-γ gene in the example to be described later) in the absence of an inducer such as IPTG. Furthermore, since the tac promoter in ptacGIF54C is directed to the β-lactamase ($Ap^r$) gene, there is the possibility of the β-lactamase protein being produced in excess as well. Therefore, as will be mentioned in detail in the example, the $laci^q$ gene was introduced into the plasmid ptacGIF54C, and a plasmid, $ptacGIF54Ci^q$, with the directionality of the tac promoter reversed therein was constructed. An Escherichia coli transformant carrying this plasmid had a hIFN-γ (antiviral) activity about 16 times higher as compared with an Escherichia coli transformant carrying pGIF54C (cf. Table 2).

Furthermore, the present inventors made an attempt to accomplish high production of useful polypeptides by introducing the runaway plasmid DNA said to cause rapid increase in plasmid copy number at elevated temperatures. The runaways plasmid used was a gift from Dr. M. Inoue at New York State University. A host Escherichia coli strain carrying said plasmid has been named W620recA/pYM307 and deposited with the Fermentation Research Institute, Agency of Industrial Science and Technology, Japan, under the deposit number FERM BP-345. pYM307 is derived from the runaway plasmid described by B. E. Uhlin et al. in Gene, 6, 91, 1979. This runaway plasmid of Uhlin et al. is now in wide use.

A host Escherichia coli strain (WA802/pY tacGIF) transformed with the plasmid pY-tacGIF constructed by introduction of a runaway plasmid DNA fragmnet was found to have an antiviral (hIFN-γ) activity about 16 times still higher as compared with an Escherichia coli transformant carrying ptacGIF54Ci$^q$, as shown later in the example.

In this way, the present inventors, as a result of intensive study, succeeded in constructing high expression vectors more suited for efficient production of useful polypeptides than expected.

The present invention, which has been completed on the basis of the above findings, provides an Escherichia coli plasmid which has, between the Shine-Dalgarno sequence and the translation start codon, a base sequence different from the corresponding, originally occurring region in the β-galactosidase oepron and contains, upstream therefrom, the region of a hybrid promoter between the Escherichia coli trp promoter and the lac promoter and, at an entirely independent site, a lac promoter repressor gene; a host procaryotic cell transformed with a plasmid containing a gene coding for the amino acid sequence of a useful polypeptide as inserted therein so that said gene can be expressed under the control of the above hybrid promoter; and a culture containing a polypeptide having the amino acid sequence of human immune interferon as obtained by cultivating a transformant selected from the group consisting of WA802/ptacGIF54Ci$^q$ and WA802/pYtacGIF.

In the accompanying drawings, Fig. 1 schematically illustrates the process for the construction of the plasmids pGIF54A and pGIF54C;

Fig. 2 schematically illustrates the process for the construction of the plasmid ptacGIF54C;

Fig. 3 schematically illustrates the process for the construction of the plasmid ptacGIF54Ci$^q$;

Fig. 4 is a schematic representation of the restriction enzyme map of the plasmid pINIII containing the lac promoter repressor gene i$^q$;

Fig. 5 schematically illustrates the process for the construction of the plasmid pY-tacGIF;

Fig. 6 is a schematic representation of the restriction enzyme map of the runaway plasmid; and

Fig. 7 shows the amino acid sequence of hIFN-γ and the base sequence of a chemically synthesized, hIFN-γ-encoding gene.

The following examples illustrate the invention. For the sake of brevity, the utility of the plasmids according to the invention is shown by giving an example of hIFN-γ polypeptide production. It is obvious that the plasmids are also applicable to the production of other useful polypeptides.

## Examples

1. Construction of the plasmids pGIF54A and pGIF54C (cf. Fig. 1) and antiviral activity

   A. Construction of pGIF54A and pGIF54C

   The plasmid pGIF54 was once used for transforming the deoxycytosine-methylation deficient (dcm⁻) Escherichia coli strain GM271 (provided by the E. coli Genetic Stock Center at the Department of Human Genetics, Yale University School of Medicine, New Haven, Connecticut, USA; CGSC strain No. 6477). A 5 µg portion of the pGIF54 plasmid DNA recovered from the transformant was dissolved in 50 µl of SalI solution (10 mM Tris hydrochloride buffer, pH 7.4, containing 10 mM $MgCl_2$, 150 mM NaCl and 10 mM mercaptoethanol). Following addition of 20 units of the restriction enzyme SalI and 10 units of PstI, digestion was effected at 37°C for 1 hour. The reaction mixture was subjected to agarose gel electrophoresis, and a PstI-SalI fragment comprising about 1300 base pairs was electrophoretically separated, treated with phenol and then precipitated by addition of alcohol. The precipitated DNA was dissolved in 50 µl of 6 mM Tris hydrochloride buffer (pH 8.0) containing 60 mM sodium chloride and 10 mM magnesium chloride, followed by addition of 10 units of AvaII. After digestion at 37°C for 1 hour, the reaction mixture was subjected to agarose gel electrophoresis.

The desired 436 base pair AVAII-SalI fragment was extracted electrophoretically, treated with phenol and precipitated by addition of alcohol.

Separately, 2 µg of pGIF54 DNA was dissolved in 50 µl of SalI solution and, following addition of 6 units of EcoRI and 10 units of SalI, digestion was effected at 37°C for 1 hour. The reaction mixture was subjected to gel agarose electrophoresis and the desired EcoRI-SalI fragment comprising about 3800 base pairs was extracted electrophoretically, treated with phenol and precipitated by addition of alcohol.

Furthermore, two DNA fragments, (A) and (C), having the respective base sequences shown in Fig. 1 and EcoRI and AvaII cohesive ends were synthesized by the solid method disclosed in Japanese Patent Application No. 86180/1982. A 20 pmol portion of each oligonucleotide was dissolved in 10 µl of 66 mM Tris hydrochloride buffer (pH 7.6) containing 6.6 mM magnesium chloride. Following addition of 1 µl of 4 mM ATP and 2 units of polynucleotide kinase, the phosphorylation of 5' OH was allowed to proceed at 37°C for 30 minutes.

Then, the above 3 DNA fragmens were subjected to ligation. Thus, the 436 base pair AvaII-SalI fragment and about 3800 base pair EcoRI-SalI fragment prepared in the above manner were dissolved in 10 µl of ligation solution (20 mM Tris hydrochloride buffer, pH 7.6, 10

mM $MgCl_2$). For constructing pGIF54A, 10 μl of the solution of a synthetic fragment (A) with the 5' OH phosphorylated or, for constructing pGIF54C, 10 μl of a solution of the synthetic fragment (C) with the 5' OH phosphorylated was admixed with the above solution. Following addition of 1 unit of T4 DNA ligase, the ligation reaction was carried out at 16°C overnight. The DNA solutions thus obtained were used for transformation of Escherichia coli WA802 by the conventional method. Transformant selection was conducted using a nutrient agar medium containing 40 μg/ml of ampicillin, and a number of ampicillin resistant transformants (WA802/pGIF54A and WA802/pGIF54C) were obtained. Isolation of the plasmid DNA and analysis with restriction enzymes confirmed that these transformants carried the contemplated plasmid pGIF54A or pGIF54C.

B. Assay of the transformants for antiviral activity

To quantify the expression of hIFN-γ, the parent host strain WA802/pGIF54 and the above transformants WA802/pGIF54A and WA802/pGIF54C each was cultivated at 37°C overnight in 2 ml of L-broth (containing 40 μg/ml of ampicillin). A 0.2-ml portion of each culture thus prepared was inoculated into 5 ml of the same medium as above. After incubation at 37°C until an $OD_{660}$ value of about 0.7, 0.05 ml of 100 mM IPTG was added and incubation was continued at 37°C for 2 hours. The culture (1 ml) was centrifuged at 10,000 rpm for 5 minutes and the cells obtained were subjected to lysis by adding 500 μl of 0.15 M phosphate buffer (pH 7.2) containing 1 mg/ml of lysozyme and 50 mM sodium chloride and allowing to stand in an ice bath for 10 minutes. The cells were then disrupted by 3 repetitions of rapid freezing in dry ice-ethanol and rapid thawing in a constant-temperature vessel maintained at 37°C. After centrifugation at 10,000 rpm for 10 minutes, the supernatant was assayed for antiviral activity by the method disclosed in Japanese Patent Application No. 86180/1982. The results thus obtained are shown in Table 1.

Table 1

Antiviral activity of each transformant

| Transformant | Antiviral activity (IU/ml) | Ratio |
|---|---|---|
| WA802/pGIF54 | 13.7 | 1.0 |
| WA802/pGIF54A | 48.2 | 3.5 |
| WA802/pGIF54C | 108.2 | 8.0 |

As shown in Table 1, WA802/pGIF54A and WA802/pGIF54C showed antiviral activity 3.5 times and 8.0 times stronger as compared with the original transformant WA802/pGIF54. It was thus noted that the base sequence between the SD sequence and the translation start codon has a great influence on the

expression of heterologous polypeptides (in this example, hIFN-γ). The distance between the SD sequence and the translation start codon is longer by one base pair (T-A) in pGIF54A and shorter by one base pair (C-G) in pGIF54C as compared with the plasmid before modification, namely pGIF54.

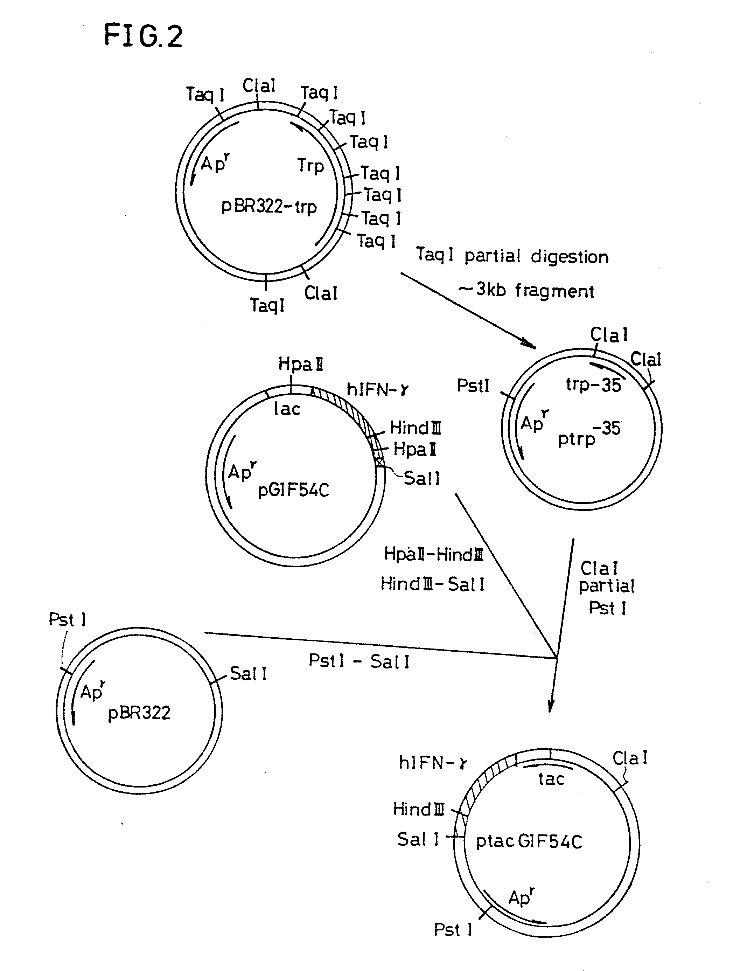

2. Modification of the promoter region: construction of the plasmid ptacGIF54C (cf. Fig. 2)

Fig. 2 is a schematic representation of the construction of the plasmid ptacGIF54C by modifying the promoter region on the plasmid pGIF54C.

In Fig. 2, the plasmid pBR322-trp is a plasmid reconstructed, in the following manner, from the plasmid pSC101-trp obtained by the method described in Gene, 1, 141-152, 1977. Thus, the pSC101-trp DNA was cleaved with HindIII, and a 4.5 Kb DNA fragment containing the Escherichia coli tryptophan promoter (trp promoter) was separated and partially digested with HpaI and an about 2.8 Kb DNA fragment was thereby isolated. This DNA fragment was ligated with the longer DNA fragment obtained by digestion of pBR322 with HindIII-PvuII, whereby pBR322-trp was constructed. A pBR322-trp-carrying Escherichia coli transformant has been deposited with the Institute for Fermentation, Osaka under the deposit number IFO-14281 and is readily available.

In the trp promoter, a TaqI site occurs between the -35 region and the Pribnow box. In the lac promoter, a HpaII site occurs at the same place. The protruding terminus remaining after cleavage with these two restriction enzymes is the same as $^{5'}CG^{3'}$. This facilitates ligation. By utilizing these cleavage sites, that portion of the trp promoter which is upstream from the TaqI site (containing the -35 region) and that portion of the lac promoter which is downstream from the HpaII site (containing the Pribnow box and the subsequent operator region) were ligated together. The details are described below.

A. Construction of the plasmid ptrp$^{-35}$

The plasmid pBR322-trp (2 μg) containing the structure covering the trp promoter, trp leader, trpE and part of trpD was dissolved in 50 μl of 33 mM Tris acetate buffer (pH 7.6) containing 66 mM potassium acetate, 10 mM magnesium acetate and 0.5 mM dithiothreitol (hereinafter, TA solution). Following addition of 2 units of TaqI, digestion was effected at 65°C for 5 minutes. Under these conditions, the cleavage with TaqI is incomplete, hence there occurred partial digestion. The desired DNA fragment is the about 3000 base pair DNA fragment resulting from cleavage of the trp promoter at the TaqI site between the -35 region and the Pribnow box and of pBR322 at the ClaI site followed by joining. Such manner of joining

allows the ClaI site to remain, which is convenient for the subsequent ligation. For isolating this DNA fragment, the partially TaqI-digested DNA was subjected to agarose gel electrophoresis, an about 3000 base pair DNA fraction was extracted electrophoretically, treated with phenol and precipitated by addition of ethanol. The precipitate thus obtained was dried and dissolved in 20 µl of 66 mM Tris hydrochloride buffer, pH 7.6, containing 6.6 mM magnesium chloride, 10 mM dithiothreitol and 0.4 mM adenosine triphosphate (hereinafter, ligation solution). Using 1 unit of T4 DNA ligase, ligation was effected at 16°C overnight. To the reaction mixture was added 2.5 volumes of ethanol, whereby a DNA precipitate was obtained. This was used for transforming Escherichia coli WA802. Transformant selection was conducted on a nutrient agar medium containing 40 µg/ml of ampicillin. Plasmid DNA separation of a number of ampicillin resistant transformants followed by plasmid checking using restriction enzymes gave the desired plasmid ptrp$^{-35}$.

B. Construction of ptacGIF54C

In the above-constructed ptrp$^{-35}$, the base just prior to the TaqI site downstream from the -35 region of the trp promoter is A and there occurs ATCGA. This is in connection with the ClaI site (ATCGAT) of pBR322. Therefore, there remains the ClaI site. By utilizing this ClaI site, ptrp$^{-35}$ can be joined to the HpaII site of the lac promoter. This ptrp$^{-35}$ has two ClaI sites, so that partial ClaI digestion is required. Accordingly, 2 µg of the ptrp$^{-35}$ DNA was dissolved in 50 µl of TA solution, 6 units of PstI was added and digestion was effected at 37°C for 10 minutes. The enzyme was then inactivated by heating the reaction mixture at 65°C for 15 minutes, and the mixture was subjected to agarose gel electrophoresis. The desired about 2300 base pair DNA fragment was extracted electrophoretically, treated with phenol and precipitated by addition of ethanol.

In the next place, for use in joining the structure from the Pribnow box and thereon of the lac promoter to the above ptrp$^{-35}$, 5 µg of the pGIF54C plasmid DNA capable of hIFN-γ expression under the control of the lac promoter was dissolved in TA solution, HindIII and HpaII (10 units each) were added, and digestion was effected at 37°C for 1 hour. The subsequent agarose gel electrophoresis separated the desired DNA fragment covering 352 base pairs from the HpaII site within the lac promoter to the HindIII site within the hIFN-γ gene. This fragment was extracted electrophoretically, treated with phenol and precipitated by addition of ethanol.

Then, to separate that structural gene portion subsequent to the HindIII site of the hIFN-γ gene, 5 µg of the same plasmid as above, i.e. pGIF54C, was dissolved in 50 µl of 10 mM Tris hydrochloride buffer, pH 7.4, containing 10 mM magnesium chloride, 150 mM sodium chloride and 10 mM 2-mercaptoethanol (hereinafter, SalI solution), 10 units of HindIII and 10 units of SalI were added, and digestion was effected at 37°C for 1 hour. The reaction mixture was subjected to agarose gel electrophoresis, and the desired 155 base pair DNA fragment was extracted electrophoretically, treated with phenol and precipitated by addition of ethanol. Furthermore, 2 µg of the pBR322 DNA was dissolved in 50 µl of SalI solution, 6 units of PstI and 6 units of SlaI were added, digestion was effected at 37°C for 1 hour, and the reaction mixture was subjected to agarose gel electrophoresis. The desired 1404 base pair DNA fragment thus separated was extracted electrophoretically, treated with phenol and precipitated by addition of ethanol. The four DNA fragments thus obtained, namely the ClaI-PstI DNA fragment (about 2300 base pairs) containing the -35 region of the trp promoter, the HpaII-HindIII DNA fragment covering 352 base pairs from the Pribnow box of the lac promoter and thereon to about two thirds of the hIFN-γ gene, the 155 base pair HindIII-SalI DNA fragment covering the remaining one third of the hIFN-γ gene and the 1404 base pair PstI-SalI DNA fragment, were dissolved in 20 µl of ligation solution. Using 1 unit of T4 DNA

ligase, ligation was effected at 16°C overnight. Ethanol was added to the reaction mixture, and the precipitate DNA thus obtained was used for transforming Escherichia coli WA802. The transformant (WA802/ptacGIF54C) was selected on a nutrient agar medium containing 40 µg/ml of ampicillin, whereby a number of ampicillin transformants were isolated. The plasmid DNA was separated from these transformants and analyzed with restriction enzymes. It was thus confirmed that said transformants carry the contemplated plasmid ptaGIF54C. In the above analysis, the contemplated plasmid was obtained in all the isolates (18 strains), namely almost completely.

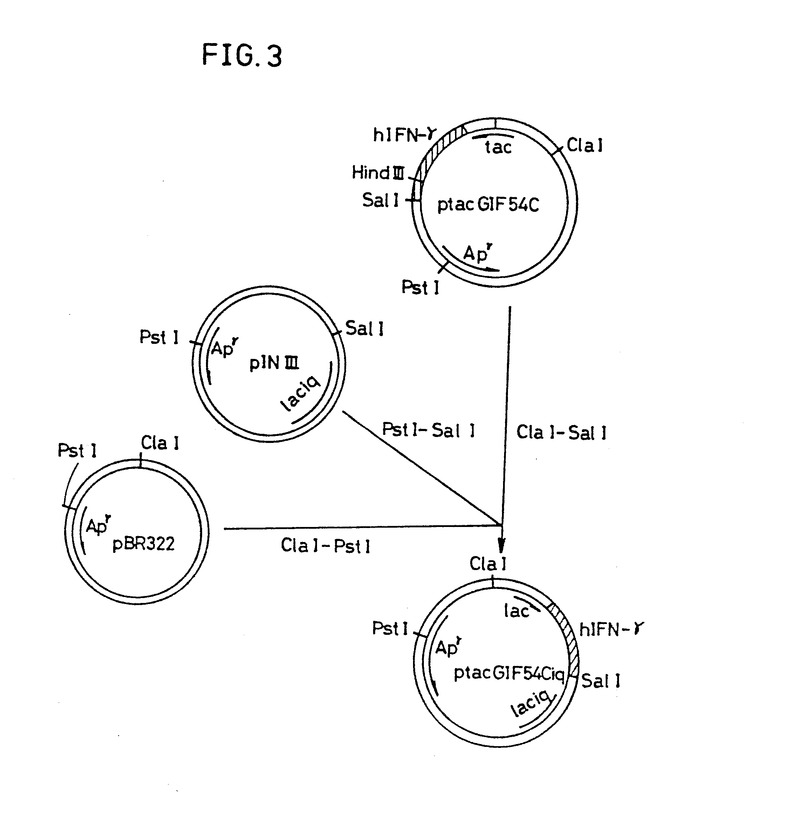

3. Construction of the plasmid ptacGIF54Ci$^q$ (cf. Fig. 3)

ptacGIF54Ci$^q$ is the plasmid constructed by introduction of the lac promoter repressor gene laci$^q$ into the plasmid ptacGIF54C obtained in Example 2-B and reversing the tac promoter region in said plasmid. Its construction is schematically illustrated in Fig. 3.

The ptacGIF54C DNA (5 µg) was dissolved in 50 µl of SalI solution, followed by addition of 12 units of ClaI and 12 units of SalI. After digestion at 37°C for 1 hour, the reaction mixture was subjected to agarose gel electrophoresis. The desired ClaI-SalI DNA fragment covering about 1000 base pairs and containing the tac promoter and the γ-interferon gene as thus separated was extracted electrophoretically, treated with phenol and precipitated with ethanol.

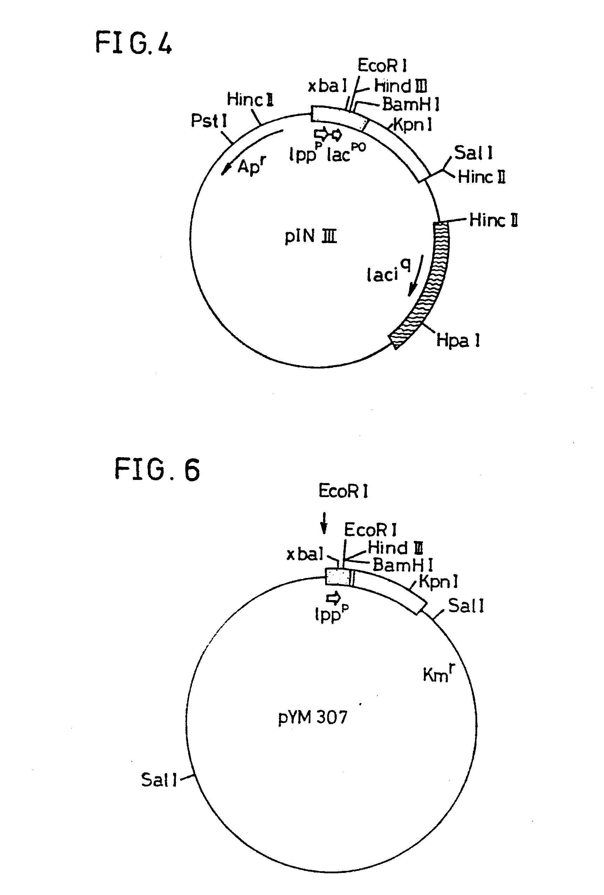

The introduction of the laci$^q$ gene was then conducted. The laci$^q$ gene-containing plasmid pINIII used in the practice of the invention, which was a gift from Dr. M. Inoue at New York State University, has the restriction enzyme map as shown in Fig. 4. An Escherichia coli transformant carrying said plasmid, JA221/pINIIIA1, has been deposited with the Fermentation Research Institute, Agency of Industrial Science and Technology, Japan, under the deposit number FERM BP-344.

The above pINIII plasmid DNA (2 µg) was dissolved in 50 µl of SalI solution, and 10 units of PstI and 10 unit of SalI were added. After digestion at 37°C for 1 hour, the reaction mixture was subjected to agarose gel electrophoresis, whereby a PstI-SalI DNA fragment covering about 5500 base pairs and containing the laci$^q$ gene was separated. Said DNA fragment was extracted electrophoretically, treated with phenol and precipitated by addition of ethanol. Furthermore, 2 µg of the pBR322 DNA was dissolved in 50 µl of TA solution, 6 units of PstI and 6 units of ClaI were added and, following digestion at 37°C for 1 hour, the mixture was subjected to agarose gel electrophoresis, whereby the desired 777 base pair PstI-ClaI DNA fragment was separated. Said DNA fragment was extracted electrophoretically, treated with phenol and precipitated by addition of ethanol.

The three DNA fragments obtained above (<u>Cla</u>I-<u>Sal</u>I and <u>Pst</u>I-<u>Sal</u>I fragments from ptacGIF54C and <u>Pst</u>I-<u>Sal</u>I fragment from pBR322) were dissolved in 20 μl of ligation solution and, using 1 unit of T4 DNA ligase, ligation was effected at 16°C overnight. Addition of ethanol to the reaction mixture gave a DNA precipitate. Using this DNA, <u>Escherichia coli</u> WA802 was transformed. Selection of the transformant (WA802/ptacGIF54Ci$^q$) was conducted on a nutrient agar medium containing 40 μg/ml of ampicillin. A number of ampicillin resistant transformants were obtained. Plasmid DNA separation and analysis using restriction enzymes confirmed that 2 out of 5 such transformants carried the contemplated plasmid ptacGIF54Ci$^q$.

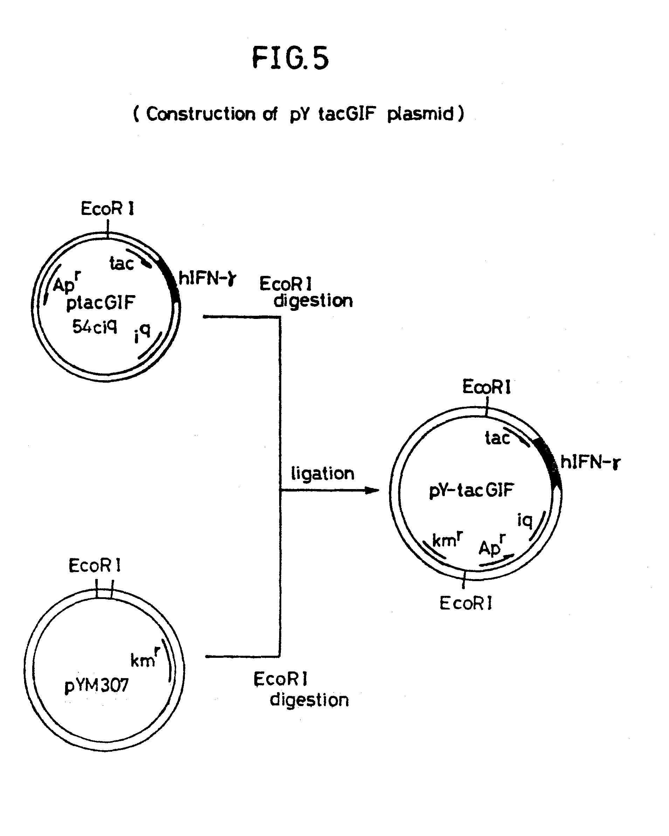

4. Introduction of the runaway plasmid DNA: construction of the plasmid pY-tacGIF (cf. Fig. 5)

The introduction of the runaway plasmid DNA into the plasmid ptacGIF54Ci$^q$ is schematicaly illustrated in Fig. 5. The <u>Escherichia coli</u> strain pYM307 carrying the runaway plasmid (cf. Fig. 6) was a gift from Dr. M. Inoue at New York State University.

The ptacGIF54Ci$^q$ DNA (2 μg) and 2 μg of the pYM307 runaway plasmid DNA were each dissolved in 50 μl of TA solution, and 12 units of <u>Eco</u>RI was added. After 1 hour of reaction at 37°C, the enzyme was inactivated by heating at 65°C for 15 minutes. Addition of ethanol gave a DNA precipitate. After drying, each DNA precipitate was dissolved in 10 µl of ligation solution. The two solutions were mixed to make 20 µl and, following addition of 1 unit of T4 DNA ligase, ligation was effected at 16°C overnight. Addition of ethanol to the reaction mixture gave a DNA precipitate, which was used for transforming Escherichia coli WA802. Since the pYM307 runaway plasmid has the kanamycin resistance gene and ptacGIF54Ci$^q$ has the ampicillin resistance, gene, the transformant (WA802/pY-tacGIF) carrying the contemplated plasmid composed of both the above plasmids joined together must exhibit both kanamycin resistance and ampicillin resistance. Therefore, transformant selection was carried out on a nutrient agar medium containing 40 µg/ml of ampicillin and 20 µg/ml of kanamycin. Since the runaway plasmid is temperature-sensitive, the transformation was conducted at 28°C. From a number of transformants thus obtained, plasmids were isolated and analyzed using restriction enzymes, and it was confirmed that 3 out of 6 such transformants carried the contemplated plasmid pY-tacGIF.

5. Assay of transformants for antiviral activity

To quantify the hIFN-γ expression in the transformants WA802/pY-tacGIF, WA802/ptacGIF54Ci$^q$ and WA802/pGIF54C, each transformant (one strain selected from each group of transformants carrying each contemplated plasmid) was grown in 2 ml of L-broth containing 40 µg/ml of ampicillin at 28°C overnight, and 0.2 ml of the culture was inoculated into 4 ml of L-broth having the same composition as above. After cultivation at 32°C until an $OD_{660}$ of about 0.7, 0.2 ml of 10 mM IPTG was added and the incubation was continued at 37°C for further 3 hours.

Bacterial cells were collected by centrifuging 1 ml of the above culture broth at 10,000 rpm for 5 minutes, 500 µl of 0.15 M phosphate buffer (pH 7.2) containing 1 mg/ml lysozyme and 50 mM sodium chloride was added thereto and the mixture was allowed to stand on an ice bath for 10 minutes to thereby cause lysis. Thereafter, rapid freezing in dry ice-ethanol followed by rapid thawing in a constant-temperature bath maintained at 37°C was repeated three times to thereby cause cell disruption, followed by centrifugation at 10,000 rpm for 10 minutes. The supernatant was assayed for antiviral activity by the method disclosed in Japanese Patent Application Kokai Tokkyo Koho No. 86180/1982. The results thus obtained are shown in Table 2.

0136829

- 24 -

## Table 2

### Antiviral activity of each transformant

| Transformant | Antiviral activity (IU/ml) | Ratio |
|---|---|---|
| WA802/pGIF54C | 74 | 1.0 |
| WA802/ptacGIF54Ci$^q$ | 1,170 | 15.8 |
| WA802/pY-tacGIF | 18,724 | 253.0 |

As shown in Table 2, the plasmid ptacGIF54Ci$^q$ constructed by introduction of the new promoter (tac promoter) and the lac promoter repressor gene into the lac-dependent expression vector pGIF54C brought about an antiviral activity about 16 times higher as compared with pGIF54C, and the plasmid pY-tacGIF constructed by further introduction of the runaway plasmid DNA brought about an antiviral activity still 16 times higher as compared with ptacGIF54Ci$^q$ (253 times higher as compared with the original pGIF54C). As compared with the starting plasmid pGIF54, the final plasmid pY-tacGIF is thus about 2,000 times higher in antiviral activity production capacity. This indicates that the improvement in the plasmid vector in accordance with the present invention are surprisingly effective in the production of heterologous polypeptides.

The antiviral activity shown in Table 2 disappeared upon treatment at pH 2 and upon neutralization with an hIFN-γ-specific antibody. Said activity, therefore, undoubtedly reflects the hIFN-γ production. Although there have been disclosed various methods of interferon production using the rDNA technology, it is for the first time that such novel vectors that enable high production of useful polypeptides have actually been constructed as a result of intensive plasmid modification efforts used by the present inventors.

Claims:

1. An Escherichia coli plasmid which has, between the Shine-Dalgarno sequence and the translation initiator codon, a base sequence different from the corresponding, originally occurring region in the β-galactosidase operon and contains, upstream therefrom, the region of a hybrid promoter between the Escherichia coli trp promoter and the lac promoter and, at an entirely independent site, a lac promoter repressor gene.

2. A plasmid as claimed in claim 1, wherein a plasmid DNA capable of causing an increase in plasmid copy number (runaway plasmid DNA) is inserted therein.

3. A plasmid as claimed in claim 1 or 2, wherein a gene coding for the amino acid sequence of a useful polypeptide is inserted therein so that said gene can be expressed under the control of said hybrid promoter.

4. A plasmid as claimed in claim 3, wherein said useful polypeptide is human immune iterferon.

5. A plasmid as claimed in claim 4, wherein the gene coding for the amino acid sequence of human immune interferon is a chemically synthesized gene or a gene prepared from an mRNA isolated and purified from the human spleen tissue or peripherial blood lymphocytes.

6. A plasmid as claimed in claim 5, wherein the chemically synthesized gene has the base sequence given in Fig. 7.

7. The plasmid of any of claims 1 and 3-6, which is selected from the group consisting of ptacGIF54Ci$^q$ plasmid species.

8. The plasmid of any of claims 2-6, which is selected from the group consisting of pY-tacGIF plasmid species.

9. A host procaryotic cell transformed with the plasmid of any of claims 3-8.

10. A transformant as claimed in claim 9, wherein the host procaryotic cell is of Escherichia coli.

11. A transformant as claimed in claim 9, which is selected from the group consisting of WA802/ptacGIF54Ci$^q$ and WA802/pYtacGIF.

12. A culture containing a polypeptide having the amino acid sequence of human immune interferon as obtained by cultivating the transformant of claim 11.

# FIG.1

Construction of pGIF54A & pGIF54C

(A)
5′ AATTCTATGTGCTAGTGCCAG
GATACACGATCACGGTCCTG

(C)
5′ AATTATGTGCTACTGCCAG
TACACGATGACGGTCCTG

chemically synthesized
DNA fragment
(A) or (C)

FIG.2

FIG.3

FIG.4

pIN III

FIG.6

pYM 307

FIG.5

( Construction of pY tacGIF plasmid )

# FIG.7

```
         1                                              10                                                        20
Met Cys Tyr Cys Gln Asp Pro Tyr Val Lys Glu Ala Glu Asn Leu Lys Lys Tyr Phe Asn Ala Gly His Ser Asp
5' AATTC ATG TGC TAC TGC CAG GAC CCA TAC GTG AAG GAA GCT GAA AAC CTG AAG AAA TAC TTC AAC GCT GGT CAT TCT GAC
3'   G TAC ACG ATG ACG GTC CTG GGT ATG CAC TTC CTT CGA CTT TTG GAC TTC TTT ATG AAG TTG CGA CCA GTA AGA CTG

                              30                                                    40
Val Ala Asp Asn Gly Thr Leu Phe Leu Gly Ile Leu Lys Ash Trp Lys Glu Glu Ser Asp Arg Lys Ile Met Gln
GTT GCT GAC AAC GGT ACT CTG TTC CTG GGT ATC CTG AAA AAC TGG AAA GAA GAA TCT GAC CGT AAA ATC ATG CAG
CAA CGA CTG TTG CCA TGA GAC AAG GAC CCA TAG GAC TTT TTG ACC TTT CTT CTT AGA CTG GCA TTT TAG TAC GTC
 50                                             60                                                      70
Ser Gln Ile Val Ser Phe Tyr Fhe Lys Leu Phe Lys Asn Phe Lys Asp Asp Gln Ser Ile Gln Lys Ser Val Glu
TCT CAG ATC GTT TCT TTC TAC TTC AAG CTG TTC AAA AAC TTC AAG GAC GAC CAG TCT ATC CAG AAA TCT GTT GAA
AGA GTC TAG CAA AGA AAG ATG AAG TTC GAC AAG TTT TTG AAG TTC CTG CTG GTC AGA TAG GTC TTT AGA CAA CTT

Thr Ile Lys Glu Asp Met Asn Val Lys Phe Phe Asn Ser Asn Lys Lys Lys Arg Asp Asp Phe Glu Lys Leu The
ACT ATC AAG GAA GAC ATG AAC GTT AAG TTC TTC AAC TCT AAC AAG AAA AAG CGT GAC GAC TTC GAA AAG CTT ACT
TGA TAG TTC CTT CTG TAC TTG CAA TTC AAG AAG TTG AGA TTG TTC TTT TTC GCA CTG CTG AAG CTT TTC GAA TGA
100                                             110                                                    120
Asn Tyr Ser Val Thr Asp Leu Asn Val Gln Arg Lys Ala Ile His Glu Leu Ile Gln Val Met Ala Glu Leu Ser
AAC TAC TCT GTT ACT GAC CTT AAT GTA CAG CGT AAA GCT ATC CAT GAA CTG ATC CAG GTT ATG GCT GAA CTG TCC
TTG ATG AGA CAA TGA CTG GAA TTA CAT GTC GCA TTT CGA TAC GTA CTT GAC TAG GTC CAA TAC CGA CTT GAC AGG
                    130                                              140                      146
Pro Ala Ala Lys Thr Gly Lys Arg Lys Arg Ser Gln Met Leu Phe Arg Gly Arg Arg Ala Ser Gln Slp
CCG GCT GCT AAA ACT GGT AAG CGT AAA AGA TCT CAG ATG CTG TTC CGT GGT CGT CGT GCT TCT CAG TAA G  3'
GGC CGA CGA TTT TGA CCA TTC GCA TTT TCT AGA GTC TAC GAC AAG GCA CCA GCA GCA CGA AGA GTC ATT CAGCT 5'
```